# NEW EUROPEAN PATENT SPECIFICATION

(11) **EP 0 588 578 B2**
(45) Date of publication and mention of the opposition decision: **13.04.2005**
(45) Mention of the grant of the patent: 08.12.1999
(21) Application number: 93307185.4
(22) Date of filing: 13.09.1993
(51) Int. Cl.: A61K 39/00, A61K 39/145, A61K 39/02

(54) **Composition containing both a soluble and an insoluble form of an immunogen**
Zusammensetzung, die sowohl eine lösliche als auch eine unlösliche Form eines Immunogens beinhaltet
Composition contenant à la fois une forme soluble et une forme insoluble d'un immunogène

(30) Priority: 14.09.1992 US 943173
(43) Date of publication of application: 23.03.1994
(73) Proprietor: CONNAUGHT LABORATORIES INCORPORATED, Swiftwater Pennsylvania 18370-0187 (US)
(72) Inventor: Becker, Robert S., Henryville, Pennsylvania 18332 (US); Biscardi, Karen, South Sterling, Pennsylvania 18464 (US); Ferguson, Laura, Bethlehem, Pennsylvania 18017 (US); Erdile, Lorne, Stroudsberg, Pennsylvania 18360 (US)
(74) Representative: Weiss, Wolfgang, Dipl.-Chem. Dr.

(56) References cited:
- EP-A- 0 270 295
- WO-A-91/14449
- WO-A-92/13002
- WO-A-93/08299
- US-A- 4 496 538
- US-A- 4 619 828
- US-A- 4 950 480
- CHEMICAL ABSTRACTS, vol. 119, no. 5, August 02, 1993, Columbus, Ohio, USA C.K. STOVER et al. "Protective immunity elicited by recombinant Bacille Calmette-Guerin (BCG) expressing outer surface protein A (OspA) lipoprotein: A candidate Lyme disease vaccine." pages 763-7, abstract-no. 47 100y & J. EXP. Med. 1993, 178(1), 197-209
- Webster et al, J. Immonology,1977, vol.119(6), p.2073-2077

## Description

The present invention relates to vaccination and, in particular, to formulating vaccines so as to achieve an enhanced immunogenic response to an antigen. The invention also relates to the generation of antibodies in animals for use in experimental, diagnostic and therapeutic work.

Vaccination is a procedure whereby an immune response to an antigen can be achieved to protect a host from infection. Some antigens elicit a strong immune response and some a weak response. Attempts have been made to enhance the immune response of weakly immunogenic materials. The use of certain chemical adjuvants in animals has resulted in potentiation of the immune response. However, most of the adjuvants used suffer from the disadvantage that they are toxic and therefore cannot be used in humans.

Another way of achieving potentiation is to conjugate a weakly immunogenic material to a strongly immunogenic material and administer the resulting conjugate in a vaccine. For example, a conjugate of the capsular polysaccharide of Haemophilus influenzae type b to diphtheria toxoid, as described in U.S. Patents Nos. 4,496,538 and 4,619,828, or a conjugate of a weak antigen to a monoclonal antibody targeting antigen-presenting cells, as described in U.S. Patent No. 4,950,480, may be employed.

WO92/13002 discloses the use of two different forms of influenza virus for obtaining a synergistic effect in a vaccine, including the use of a whole inactivated virus and a soluble protein M.

The present invention provides a composition for eliciting an immune response to an antigen in an animal, comprising a first physio-chemical form of antigen favouring presentation of the antigen by B cells to T cells in the animal, and a second physio-chemical form of the antigen favouring presentation of the antigen by accessory cells to T cells in the animal, characterised in that said first physio-chemical form is a soluble form of said antigen and said second physio-chemical form is an insoluble form of said antigen and characterised in that one physio-chemical form of antigen is lipidated and the other physio-chemical form is not lipidated.

The two different physio-chemical forms of the same antigen are administered simultaneously in a naive animal to achieve the greatest degree of potentiation.

In order for the enhanced immune response to be achieved, it is necessary that the animal to which the antigen is co-administered, including humans, be naive, i.e. the animal has not previously been immunised by a highly-immunogenic form of the antigen. Co-administration of the antigen to a primed animal elicits no enhancement of immune response.

While the applicant does not wish to be bound to any specific theory to explain why the present invention is effective in achieving potentiation of an antigenic response by the simultaneous adminstration of at least two different physio-chemical forms of the antigen, the following theoretical explanation can be made.

B cells and accessory cells may have preferences for different physio-chemical forms of antigens. It is known that both B cells and accessory cells must present antigens to T-cells to initiate an antibody response to the antigen in naive animals (refs. 1, 2, 3, 4 and 5). These preferences may result from B cells and accessory cells using different mechanisms for internalising antigens.

B cells internalise antigens via specific binding to their cell surface immunoglobulins (refs. 6, 7) and can present soluble antigens in concentrations as low as 1 ng/ml (ref. 8). Accessory cells, which are macrophages and dendritic cells, on the other hand, internalise antigens by non-specific phagocytisis and pinocytosis (ref. 9). Macrophages can present soluble antigens if they are at concentrations of approximately 100 µg/ml (ref. 8). Other research (ref. 10) has demonstrated that the concentration of soluble antigen needed by macrophages can be decreased by binding the antigen to a particulate structure.

During the generation of an antibody response, B cells and accessory cells present antigens to T cells at two different stages of T cell activation/differentiation. Research has demonstrated that naive T cells must first interact with antigen presenting accessory cells to become activated helper T cells (refs. 11, 12, 13, 14). The inventors believe that particulate forms of antigens, which may be employed herein, effectively mediate the accessory cell activation of naive T cells. This interaction, however, is insufficient to induce the B cells to respond to a T cell-dependent antigen. Direct cell-to-cell contact between B cells and activated helper T cells is required for the induction of antibody secretion from B cells (ref. 15). This interaction is mediated by B cells processing and presenting the antigen to activated T cell (ref. 16). This type of B cell-T cell interaction is termed cognate T cell help. The inventors believe that a soluble form of antigen, which may be employed herein, best mediates B cells interacting with helper T cells.

The requirement to simultaneously administer the two physio-chemical forms of the antigen at the same injections site may have some correlation with the finding that the order of the two interactions is important in bringing about the required immune response in naive T cells. It has recently been shown that antigen presentation of B cells to naive T cells induces T cell tolerance rather than activation (refs. 2, 3). Optimal immune response appears to require antigen presentation by both B cell and accessory cells.

One physio-chemical form of the antigen is soluble and the other physio-chemical form of the antigen is insoluble, e.g. the haemaglutinin antigen (HA) of influenza virus.

One physio-chemical form of the antigen may be a lipidated protein in which case the other physio-chemical form is a non-lipidated protein, e.g. the OspA antigen.

One physio-chemical form may be a protein with a hydrophobic region in which case the other physio-chemical form is a protein without a hydrophobic region.

At least one of the physio-chemical forms of protein antigen may have been engineered e.g. by genetic engineering or by chemical synthesis, to be provided with desired epitopes or regions or to have desired epitopes or regions removed.

The antigen may contain protective epitope(s) which result in the antigen eliciting only a weak immunogenic response.

The antigen may be a viral, bacterial, fungal, protozoan or parasite protein.

The antigen may be the haemagglutinin antigen (HA) of influenza virus or other viral proteins associated with viral membranes.

The antigen may be the outer surface protein A (OspA) of the B. burodorferi spirochete (i.e. a bacterial protein). Lipidated OspA is a strong immunogen and hence co-administration with other forms of the OspA is usually not required. However, the results presented indicate the general applicability of the procedure.

The said second form of the antigen may be derived from the first by purification and/or by modification.

The different physio-chemical forms of the antigen for co-administration may vary widely, depending on the antigen chosen and the specific antigenic forms of the antigen which might be available. Preferably through, the two forms are tailored to provide for antigen presentation both by B cells and by accessory cells to T cells to initiate antibody response.

The invention will now be described by way of Examples 1 to 4 below and with reference to Figures 1 to 9 in which:-
Figures 1 and 2 are bar charts representing haemagglutination inhibition (HAI) titers achieved by various forms of HA antigen in naive guinea pigs, as detailed in Example 1 below; Figures 3 to 5 are bar charts representing IgG anti-HA responses achieved by various forms of HA antigen in guinea pigs, as detailed in Example 2 below;
Figures 6 and 7 are bar charts representing HAI titers achieved by various forms of HA antigen in primed guinea pigs, as detailed in Example 3 below; and
Figures 8 and 9 are plots of data of the immune responses achieved by various form of OspA in naive mice, as detailed in Example 4 below.

### EXAMPLES

### Example 1: The elicitation of immune responses in guinea pigs by the co-administration of different physio-chemical forms of HA from influenza virus - detection of immune response by haemagglutination inhibition (HAI)

Several different physio-chemical forms of HA exist, namely HA(p), split HA and inactivated whole virus. HA(p) is a highly purified form of HA that has had its hydrophobic tail removed and is soluble in water. Split HA is a detergent extracted and partially purified form of the HA antigen. Inactivated whole virus is formalin inactivated whole virus particles.

Split HA and inactivated whole virus are immunogenic in naive animals and humans. HA(p) is not immunogenic in naive animals or infants, even though it is antigenic in antibody-antigen reactions.

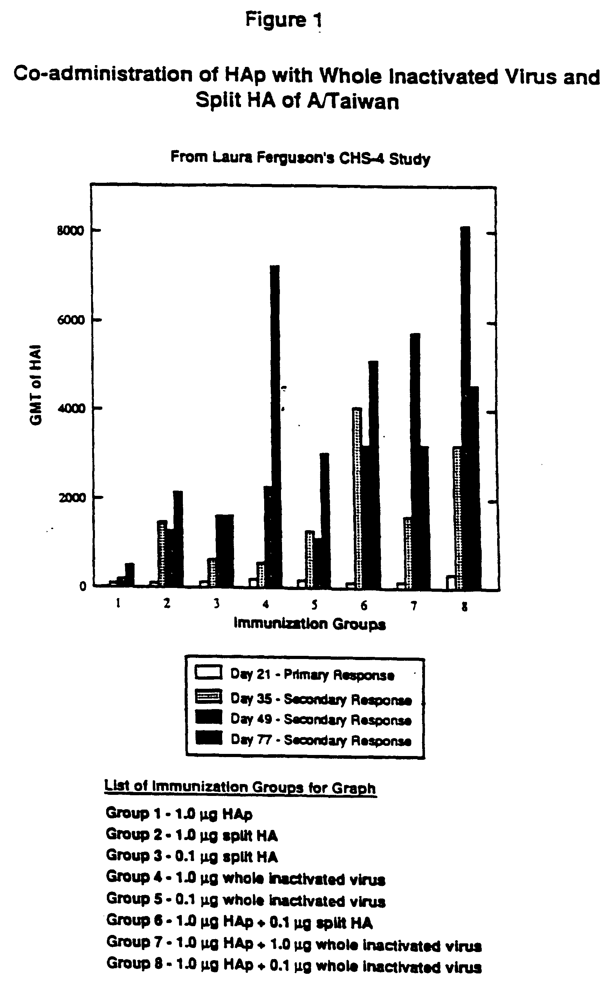

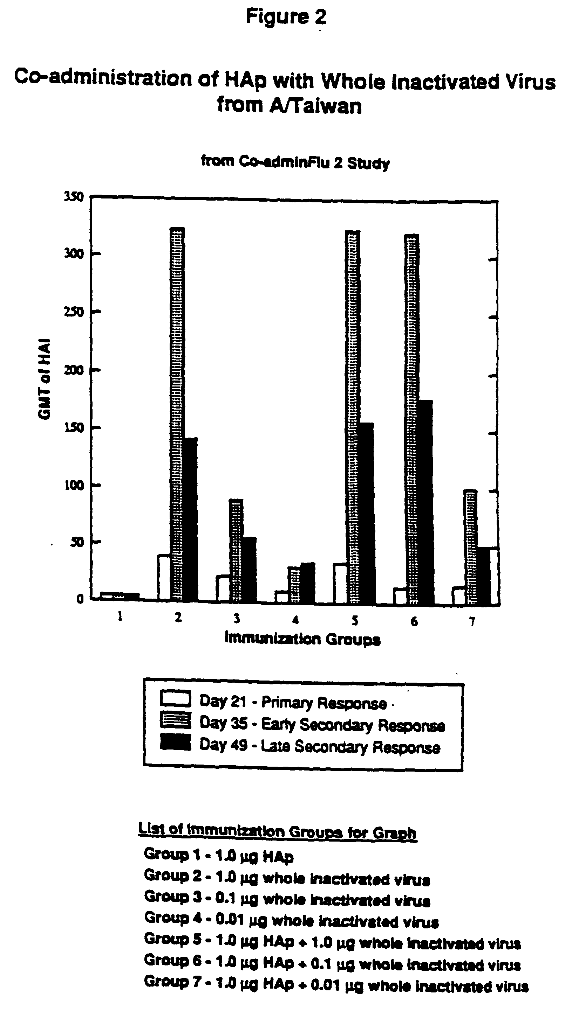

Two series of experiments were carried out in which guinea pigs were immunised with various physio-chemical forms of HA from the A/Taiwan influenza strain, alone or in combination, and their responses were measured by haemagglutination inhibition (HAI) titers, HAI titers being known to correlate well with protective immune responses. The results obtained in the experiments were plotted graphically and appear as Figures 1 and 2.

In these experiments, the amount of HA(p) was maintained constant (1.0 µg) and the amount of added whole inactivated virus was varied. Of the three amounts of whole inactivated virus employed (1.0 µg, 0.1 µg and 0.01 µg), immune responses were best potentiated by co-administration using 0.1 µg whole inactive virus, as may be seen from Figs. 1 and 2.

When the titers for this combination were compared to the titers for HA(p) or 0.1 µg whole inactive virus alone, co-administration potentiated immune responses four to seven fold at two to four weeks after the boost. At the higher dose of 1.0 µg of whole inactive virus, immune responses of co-administration were equal to the responses to the virus alone, again as seen in Figs. 1 and 2. At the low does of 0.01 µg whole inactivated virus, the immune response of both co-administration and whole inactivated virus alone were low (see Fig. 2). Since HAI titers correlate well with protective immune responses, these results suggest that co-administration enhances protective immune responses in guinea pigs.

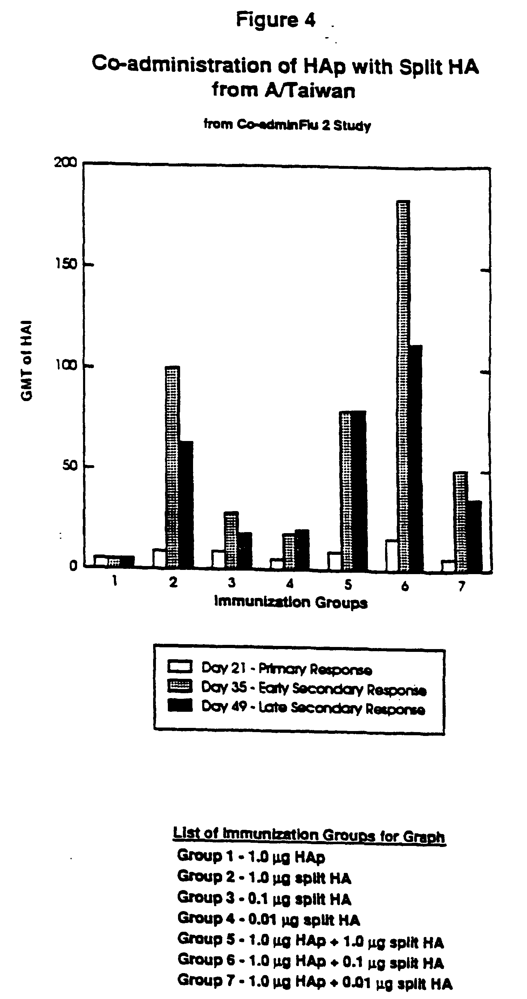

The co-administration of split HA and HA(p) also enhanced anti-HA antibody responses in guinea pigs. Maximum enhancement by co-administration was observed using 0.1 µg of HA(p) and 0.1 µg of split HA, as may be seen from the results of Figures 1 and 4. A three- to seven-fold enhancement in HAI titers was observed using these amounts of antigen.

### Example 2: Elicitation of immune responses as in Example 1 - detection and quantitation by Enzyme-linked immuno-absorbent assay (ELISA)

The antibody responses of Example 1 were also analysed by EIA (ELISA immuno-assay) to determine whether the enhancement of HAI titers by co-administration was related to the total amount of IgG anti-HA antibody generated. In these experiments, HAe (a highly-purified form of HA that retains its hydrophobic tail) was used to coat the wells of the EIA plate and anti-guinea pig IgG was used as a detecting antibody. The dilution curves of experimental antisera were compared to the dilution curve of a standard guinea pig antiserum and, on the basis of that comparison, the units of IgG anti-HA were calculated in each sera.

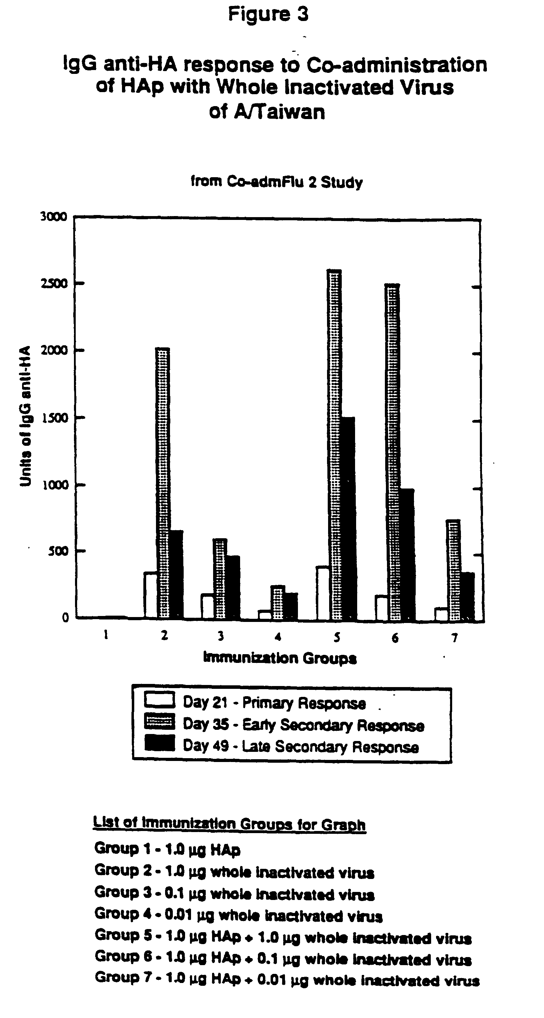

Using the same guinea pig sera, a good correlation was found when the results of the EIA, as seen in Fig. 3, were compared with the results of the HAI, as seen in Fig. 2. These results show that co-administration of the HA in different forms enhances the total amount of IgG generated against HA.

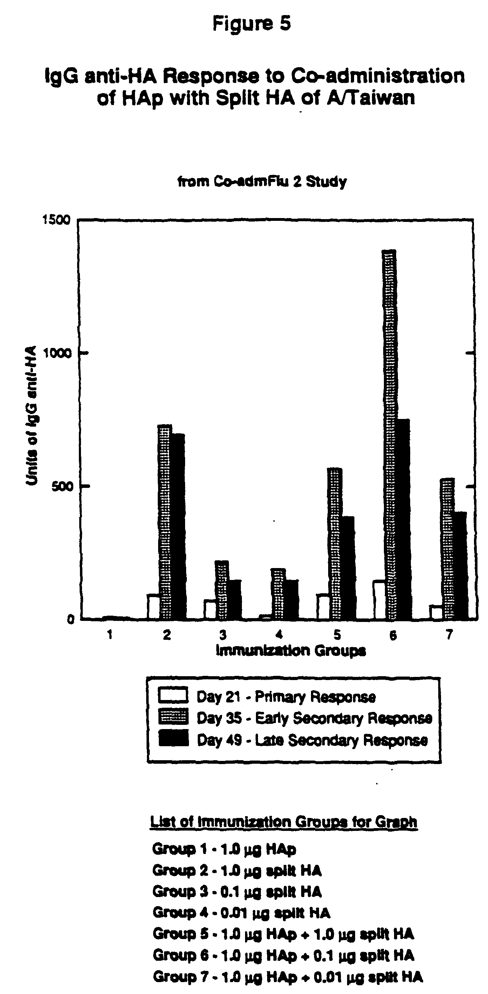

The results of EIA on sera from an experiment using split HA, as seen in Fig. 5, indicated that the increased HAI titers from co-administration were the result of increased amounts of anti-HA antibodies. From the results set forth in Examples 1 and 2, it is apparent that the levels of antibody generated to co-administration with split HA were generally less than those to co-administration with whole inactivated virus, as may be seen from Fig. 1 and a comparison of Figs. 2 and 4 and Figs. 3 and 5.

In the experiments reported in Examples 1 and 2, naive animals were used to evaluate co-adminstration.

### Example 3: The effect of co-administration of different forms of HA in primed animals

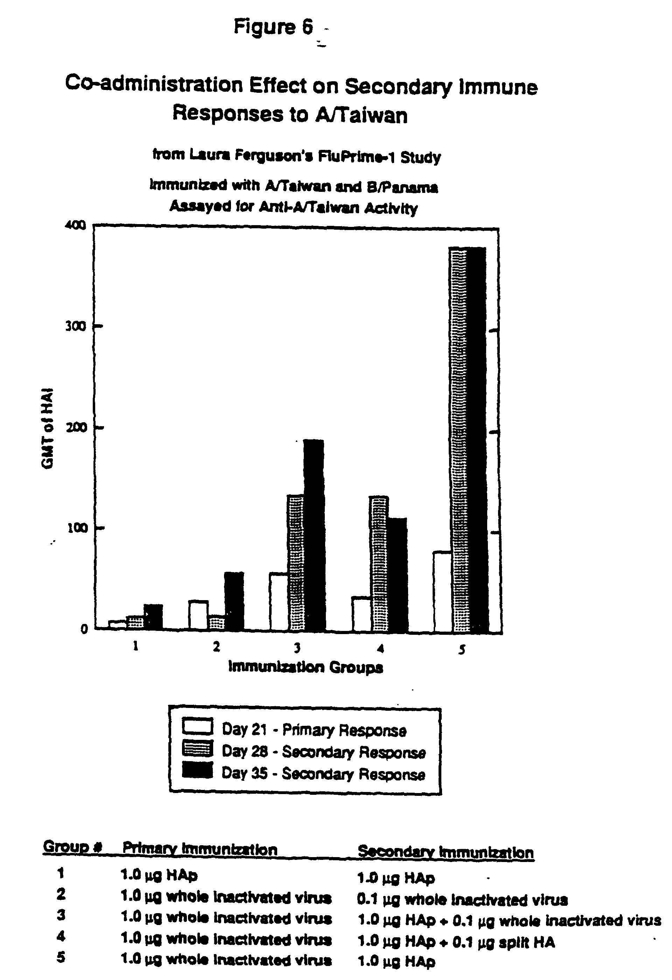

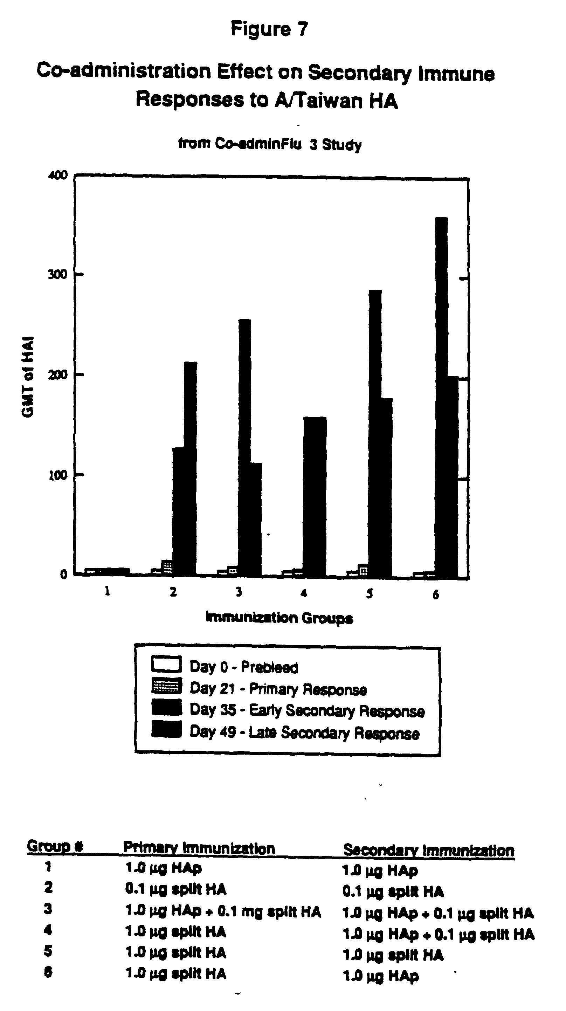

Guinea pigs were primed with either 1.0 µg of whole inactivated virus (results depicted in Fig. 6) or 1.0 µg of split HA (results depicted in Fig. 7). Three weeks later, the guinea pigs were given secondary immunisation of either single flu antigen or co-administered flu antigens. The results shown in Figs. 6 and 7 indicate that co-administration does not enhance anti-HA results in primed animals and hence the co-administration technique is useful only in naive animals, if an enhanced immune response is to be achieved.

These results also show that the superior antigen for recalling memory responses was HA(p) alone, while immunisation with HA(p) at the primary and secondary immunisation did not generate a significant immune response. These results show that HA(p) can recall memory immune responses to the HA antigen but cannot itself generate memory.

### Example 4: The elicitation of immune reactions in mice by different physio-chemical forms of the OsPA protein of B. burgdorferi spirochete

OspA lipoprotein (OSpA-L) is a very potent immunogen. Removal of the lipid moiety from OspA dramatically decreases its immunogenicity but not its antigenicity, as described in WO 93/08299.

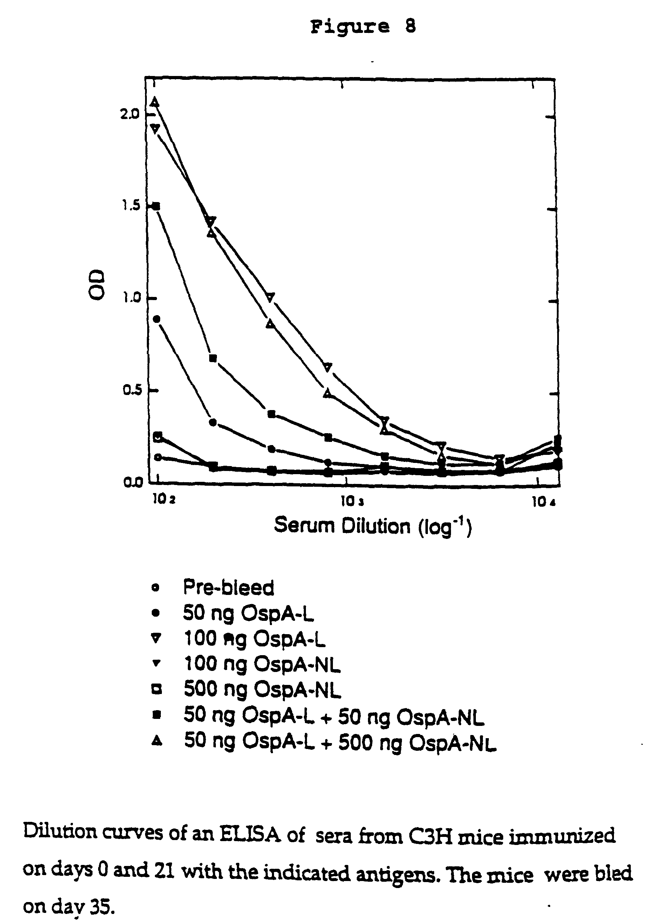

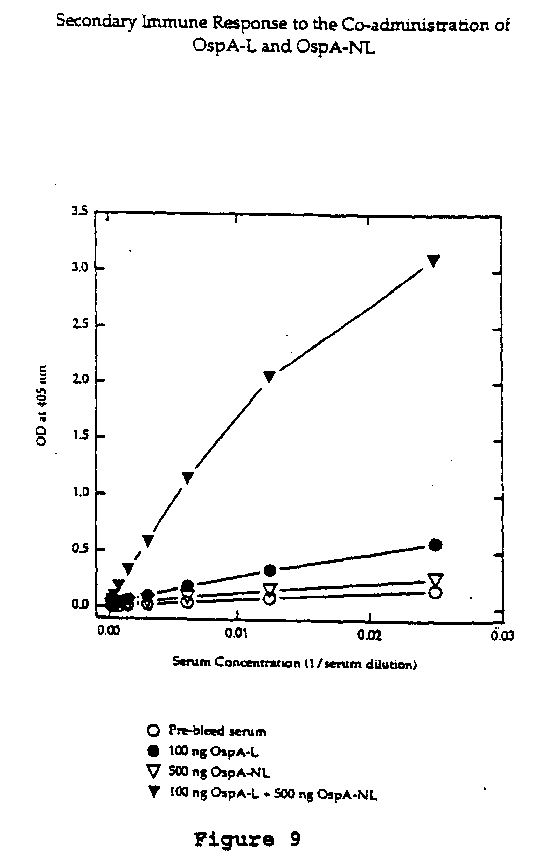

A small dose of OspA-L was co-administered to C3H/He mice with a large dose of OspA-NL and the response compared to the responses of OspA-L or OspA-NL alone. The mice were immunised at days 0 and 21 with the antigens and the mice were bled on day 35. The dilution curves of an ELISA assay of sera from the mice were plotted graphically and the results are shown in Figure 8. Immune responses are also shown in Figure 9. The data shows a potentiation of OSpA response was achieved by co-administration of OspA-L and OspA-NL relative to administration of OspA-L or OspA-NL alone.

Whilst the invention has been described particularly by way of Examples 1 to 4 above, many variations and modifications thereof are possible within the scope of the invention as defined in the appended claims.

### REFERENCES

1. "Mechanisms of T cell-B cell Interaction", Singer et al. Ann. Rev. Immunol. 1983, 1:211-41.
2. "Antigen Presentation in Acquired Immunological Tolerance", Parker et al, The FASEB Journal, Vol. 5, Oct. 1991, pp. 2771-2784.
3. "Do Small B Cells Induce Tolerance", Eynon et al, Transplantation Proceedings, Vol. 23, No 1 (February) 1991: pp. 729-730.
4. "Small B Cells as Antigen-Presenting Cells in the Induction of Tolerance to Soluble Protein Antigens" by Eynon et al, J. Exp. Med. Vol. 175, January 1992, pp. 131-138.
5. "Role of B Cell Antigen Processing and Presentation in the Humoral Immune Response", Myers, The FASEB Journal, Vol. 5, August 1991, pp. 2547-2553.
6. "Antigen Presentation by Hapten-Specific B Lymphocytes", Abbas et al, J. Immun. Vol. 135, No. 3, Sept. 1985, pp. 1661-1667.
7. "Requirements for the Processing of Antigen by Antigen-Presenting B Cells", Grey et al, J. Immun., Vol. 129, No. 6, Dec. 1982, pp. 2389-2395.
8. "Antigen-Specific B Cells Efficiently Present Low Doses of Antigen for Induction of T Cell Proliferation", Malynn et al, J. Immun. Vol. 135, No. 2, Aug. 1985, pp. 980-987.
9. "Antigen-Presenting Function of the Macrophage", Unanue, Ann. Rev. Immunol., 1985, 2: 395-428.
10. "Analysis of TX Lymphocyte Reactivity to Complex Antigen Mixtures by the Use of Proteins coupled to Latex Beads", Wirbelauer et al, Immun. Letters, 23 (1989/1990), 257-262.
11. "The Function and Interrelationships of T. Cell Receptors, Ir Genes and other Histocompatibility Gene Products", Katz et al, Transplant. Rev. (1975), Vol. 22, pp. 175-195.
12. "Restricted Helper function of F. Hybrid T Cells Positively Selected to Heterologous Erythrocytes in Irradiated Parental Strain Mice. I", Sprent, J. Exp. Med., 1978, Vol. 147, pp. 1142-1158.
13. "Restricted Helper function of F. Hybrid T Cells Positively Selected to Heterologous Erythrocytes in Irradiated Parental Strain Mice. II", Sprent, J. Exp. Med., 1978, Vol. 147, pp. 1159-1174.
14. "The Role of H-2-Linked Genes in Helper T-Cell Function", Swierkosz et al, J. Exp. Med., 1978, Vol. 147, pp. 554-570.
15. "Role of the Major Histocompatibility Complex in T Cell Activation of B Cell Subpopulations", Singer et al, J. Exp. Med., 1981, Vol. 154, pp. 501-516.
16. "Antigen-specific Interaction between T and B Cells", Lanzavecchia, Nature, Vol. 314, April 1985, pp. 537-539.

## Claims

1. A composition for eliciting an immune response to an antigen in an animal, comprising a first physio-chemical form of said antigen favouring presentation of the antigen by B cells to T cells in the animal, and a second physio-chemical form of said antigen favouring presentation of the antigen by accessory cells to T cells in the animal, **characterised in that** said first physio-chemical form is a soluble form of said antigen and said second physio-chemical form is an insoluble form of said antigen and **characterised in that** one physio-chemical form of antigen is lipidated and the other physio-chemical form is not lipidated.

2. The composition claimed in Claim 1, **characterised in that** said antigen is the haemagglutinin antigen (HA) from influenza virus, or the OspA protein.

3. The composition claimed in Claim 2, **characterised in that** said antigen is HA and the different physio-chemical forms of said antigen comprise whole inactivated virus and HA(p), or **characterised in that** the antigen is OspA of B. burgdorferi and the different physio-chemical forms of said antigen comprise OspA-L and OspA-NL.

4. The composition claimed in any one of claims 1 to 3, wherein said composition is formulated with a physiologically-acceptable carrier in a vaccine.

## Patentansprüche

1. Zusammensetzung zum Hervorrufen einer Immunantwort gegen ein Antigen in einem Tier, umfassend eine erste physiochemische Form des Antigens, die die Präsentation des Antigens durch B-Zellen gegenüber T-Zellen in dem Tier begünstigt, und eine zweite physiochemische Form des Antigens, die die Präsentation des Antigens durch Hilfszellen gegenüber T-Zellen in dem Tier begünstigt, **dadurch gekennzeichnet, dass** die erste physiochemische Form eine lösliche Form des Antigens und die zweite physiochemische Form eine unlösliche Form des Antigens ist und **dadurch gekennzeichnet, dass** eine physiochemische Form des Antigens Lipide enthält und die andere physiochemische Form keine Lipide enthält.

2. Zusammensetzung nach Anspruch 1, **dadurch gekennzeichnet, dass** das Antigen das Haemagglutininantigen (HA) des Influenzavirus oder das OspA-Protein ist.

3. Zusammensetzung nach Anspruch 2, **dadurch gekennzeichnet, dass** das Antigen HA ist und die unterschiedlichen physiochemischen Formen des Antigens das vollständige inaktivierte Virus und HA(p) umfassen, oder **dadurch gekennzeichnet, dass** das Antigen OspA von B. burgdorferi ist und die unterschiedlichen physiochemischen Formen des Antigens OspA-L und OspA-NL umfassen.

4. Zusammensetzung nach einem der Ansprüche 1 bis 3, wobei die Zusammensetzung mit einem physiologisch verträglichen Träger zu einem Impfstoff formuliert ist.

## Revendications

1. Composition pour déclencher une réaction immunitaire en réponse à un antigène chez un animal, comprenant une première forme biochimique dudit antigène favorisant la présentation de l'antigène par les lymphocytes B aux lymphocytes T chez l'animal, et une seconde forme biochimique dudit antigène favorisant la présentation de l'antigène par les cellules accessoires aux lymphocytes T chez l'animal, **caractérisée en ce que** ladite première forme biochimique est une forme soluble dudit antigène et ladite seconde forme biochimique est une forme insoluble dudit antigène et **caractérisée en ce qu'**une forme biochimique de l'antigène est lipidée et l'autre forme biochimique n'est pas lipidée.

2. Composition selon la revendication 1, **caractérisée en ce que** ledit antigène est l'antigène associé à l'hémagglutinine (HA) du virus de la grippe, ou la protéine OspA.

3. Composition selon la revendication 2, **caractérisée en ce que** ledit antigène est HA et les différentes formes biochimiques dudit antigène comprennent un virus entier inactivé et HA(p), ou **caractérisée en ce que** l'antigène est l'OspA de B. burgdorferi et les différentes formes biochimiques dudit antigène comprennent OspA-L et OspA-NL.

4. Composition selon l'une quelconque des revendications 1 à 3, dans laquelle ladite composition est formulée avec un véhicule physiologiquement acceptable dans un vaccin.
